# EUROPEAN PATENT SPECIFICATION

(11) **EP 1 044 690 B1**
(45) Date of publication and mention of the grant of the patent: **03.08.2005**
(21) Application number: 00500066.6
(22) Date of filing: 12.04.2000
(51) Int. Cl.: A61K 39/395, A61K 35/16, A61P 1/00

(54) **Applications of gamma globulin-rich plasma protein mixtures of animal origin and process for the preparation thereof**
Anwendung und Herstellungsverfahren von tierischen Gammaglobulin-reichen Plasmaprotein Mischungen
Utilisation et procédé de préparation de mélanges de protéines plasmatiques d'origine animale riches en gamma-globulines

(30) Priority: 13.04.1999 ES 9900757; 11.04.2000 ES 200000933
(43) Date of publication of application: 18.10.2000
(73) Proprietor: APC Europe S.A., 08400 Granollers (Barcelona) (ES)
(72) Inventor: Polo Pozo, Francisco Javier, 08210 Barbera del Valles (Barcelona) (ES); Rodriguez Canel, Carmen, 08016 Barcelona (ES); Rodenas Navarro, Jesus, 08031 Barcelona (ES); Saborido Martin, Maria de las Nieves, 08470 San Celoni (Barcelona) (ES)
(74) Representative: Curell Aguilà, Marcelino

(56) References cited:
- EP-A- 0 064 210
- EP-A- 0 338 229
- SU-A- 1 754 113
- US-A- 5 531 989
- HASKO F. ET AL.: "Fractionation of plasma proteins with PEG." HAEMATOLOGICA, vol. 14, no. 2, 1981, pages 199-206, XP000917117 Budapest
- PATENT ABSTRACTS OF JAPAN vol. 013, no. 169 (C-587), 21 April 1989 (1989-04-21) & JP 64 000028 A (CHEMO SERO THERAPEUT RES INST), 5 January 1989 (1989-01-05)
- PATENT ABSTRACTS OF JAPAN vol. 013, no. 556 (C-664), 11 December 1989 (1989-12-11) & JP 01 230533 A (GREEN CROSS CORP:THE), 14 September 1989 (1989-09-14)
- BPI, VFA, BAH, VAP: "Rote Liste 1999" 1999 , ECV , AULENDORF XP002144418 * see compounds 750003, 75007-750111 and 75013-75014 *
- TRUSOVA L. ET AL.: "Fractionation of blood serum proteins by non-ionic water-soluble polymers." VSES. GOS. NAUCHNO-KONTROL'N. INST. VET. PREP., 1980, pages 98-101, XP002144417

## Description

### Field of the Invention

This invention relates to applications of a mixture of plasma proteins of animal origin having a gamma globulin content of over 25 wt%, for the preparation of medicinal compositions, food and dietary preparations. It also relates to a process for the preparation of a mixture of plasma proteins of animal origin having a gamma globulin content of over 90 wt%, without the use of chromatographic columns, from a first concentrate, the gamma globulin/proteins ratio of which is over 30 wt%, said first concentrate being derived from animal plasma which has been subjected to at least one prior centrifugation operation.

### Prior Art Reference

On the basis of the known literature, the situation relating to the fractionation process is understood to be as follows:

In "*A chromatographic Procedure for the Purification of Human Plasma Albumin*". Curling, J.M., J. Berglöf, L.O. Lindquist & S. Eriksson; Vox Sang. 33:97-107 (1977), there is described a fractionation method starting out from human plasma, in which polyethylene glycol (PEG) 4000 is used to obtain a gamma globulin-rich precipitate and an albumin fraction concentrate, which is finally purified to 99% purity using a chromatographic system.

In "*A Heat Polyethylene Glycol Procedure as an Alternative Method for Plasmafractionation*", Guddat, W. & K. Hillger; Folia Haematol., Leipzig 109 (1982) 6, S. 840-855, there is described a fractionation process starting out from human plasma for preparing albumin of high purity. In the process described, fractionation steps with PEG 4000 are alternated with the use of sodium octanate, with heating to high temperatures (75°C) to obtain the albumin-enriched fraction.

In "*A new high quality Albumin for therapeutic use*", Joint WHO/IABS Symposium on the Standardization of Albumin, Plasma Substitutes and Plasmapheresis, Hansen, J.F. & M. Ezban. Geneva 1980, Develop. Biol. Standard. 48, pp. 105-112, there is described a fractionation method for the preparation of human albumin for therapeutic use in human beings, by way of injection, in which combined fractionation steps with PEG 3000 and the use of caprylic acid, temperature and ethanol are detailed, for the preparation of different plasma and high purity albumin fractions.

In "*Fractional Precipitation of Proteins with Polyethylene Glycol*", Hao, Y.L., K.C. Ingham & M. Wickerhauser, there is cited a fractionation process on human plasma for preparing different plasma fractions. According to the process described, PEG 4000 is used for a first separation, followed by purification steps in chromatographic columns.

In "*Optimization for Selective Fractionation of Bovine Blood Plasma Proteins Using Poly(ethylene glycol)*", Lee, Y.Z., T. Aishima, S. Nakai & J.S. Sim J. Agri. Food Chem. 1987, 35, 958-962, there is described a plasma fractionation process carried out on bovine plasma. By working with different PEG 4000 and sodium chloride concentrations and at certain pH values, it was possible to separate three major fractions: fibrinogen, immuno-globulins and albumin.

In "*Fractionation of Plasma with Polyethylene Glycol*", Polson, A. & Ruiz-Bravo; Vox Sang. 23. 107-118 (1972) there is described a fractionation process on human plasma using PEG 6000 for preparing different protein fractions included in the plasma. According to these workers, the process may be carried out working with PEG alone or in combination with organic salts or agents, or using chromatographic membranes.

In "*An Alternative Method of Large Scale Plasma Fractionation for the Isolation of Serum Albumin*", Schneider, W., H. Lefèvre, H. Fiedler & L.J. McCarty; Blut, Band 30, Seite 121-134 (1975), there is mentioned the possibility of obtaining high purity albumin from human plasma using sodium octanate in combination with ethanol and high temperatures (68°C) for the separation of albumin from the remaining plasma proteins. Subsequently, the albumin is precipitated with the use of PEG 4000 and after resuspension in water a liquid form is obtained, or a powder is obtained after freeze-drying.

In "*The Isolation of IgG from Mammalian Sera with the Aid of Caprylic Acid*", Steinbuch, M. & R. Audran; Archives of Biochemistry and Biophysics 134, 279-284. (1969), there is related a fractionation process on human, ovine, goat or rabbit plasma using caprylic acid (octanoic acid) for purification of the immuno-globulin-rich fraction. After desalination by dialysis, the fraction was subsequently concentrated or obtained in powder form through a freeze-drying process.

In "*Precipitation of Plasma Lipoproteins by PEG-6000 and Its Evaluation with Electrophoresis and Ultracentrifugation*", Viikari, J.; Scand. J. Clin. Lab. Invest., Vol. 36, 1976, there is related a fractionation method on human plasma using different levels of PEG 6000 for the preparation of the lipoprotein-rich (LDL, VLDL, HDL) fractions in a single precipitation step.

Finally, in "*Large Scale Preparation of Macroglobulins*"*,* Wickerhauser, M. and Y.L. Hao; Vox Sang. 23: 119-125 (1972), there is mentioned the possibility of fractionation of an immuno-globulin M-rich (IgM) fraction from the fraction III prepared from human plasma, using Cohn's aqueous alcoholic fractionating method. In this case, starting out from the precipitate III of Cohn's method and using PEG 6000 together with zinc sulphate, they obtained a supernatant which they subsequently precipitated with ammonium sulphate.

The literature covering the period from the late 80's up to date, apart from being very scarce, does not provide significantly different concepts from those expressed in the foregoing publications.

According to some of the papers cited, there is disclosed a process in which, starting out from an animal (bovine, ovine, goat or rabbit) plasma and subjecting it to a first prior centrifugation, under certain conditions, there is prepared an insolubilized fibrinogen-rich fraction and a clarified fraction containing the remaining proteins contained in the plasma and which are water soluble. After a certain treatment, this clarified fraction is subjected to a second prior centrifugation, from which there is separated a new concentrated gamma globulin-rich fraction and a new clarified albumin-rich fraction.

Certain applications of fibrinogen-rich plasma protein mixtures in human beings are known, for example in the treatment of haemophilics. Nevertheless, these applications require intravenous application, which means that the origin of the mixture has to be human, to avoid incompatibility problems.

Also known are certain applications of albumin-rich plasma protein mixtures, for example in surgery. Nevertheless, the application is also intravenous and the origin of the mixture has to be human.

### Summary of the Invention

It is an object of the invention to provide applications of a mixture of plasma proteins of porcine origin having a gamma globulin content of over 25 wt%, relative to total proteins, for the preparation of medicinal compositions, food and dietary preparations: [a] suitable for the prevention and treatment of gastrointestinal disorders in human beings; [b] suitable for the treatment of malabsorption processes in human beings; and [c] suitable for the treatment of clinical conditions accompanied by malnutrition in human beings, particularly ulcerative colitis; Crohn's disease; cancerous cachexia for chronic enteritis caused by chemo- and/or radiotherapy; infectious medical pathology involving severe malabsorption; AIDS; cystic fibrosis; low flow enterocutaneous fistulas; and infantile renal insufficiency endangering patient growth.

It is another object of the invention to provide applications of a mixture of plasma proteins of non human animal origin having a gamma globulin content of over 25 wt%, relative to total proteins, for the preparation of medicinal compositions, food and dietary preparations suitable for the treatment of a misabsorption process from the group formed by: small intestine syndrome; refractory diarrhoea of autoimmune origin; lymphoma; post-gastrectomy steatorrhea; carcinoma of the pancreas; wide pancreatic resection; mesenteric vascular insufficiency; amyloidosis; sclerodermia; and eosinophilic enteritis. In this cases the plasma proteins are preferably obtained from porcine, bovine, goat, equine and ovine plasma. The use of porcine plasma is particularly preferred, above all for the treatment of human beings.

One advantageous form of application is obtained when the medicinal compositions, food and dietary preparations are usable dissolved in water, milk, juices, yoghurts and other foodstuffs. These other foodstuffs are preferably humanised milk, paps and other baby foods.

Preferably any variety of fibres, prebiotic, probiotic products, vitamins and minerals are added to the medicinal preparations, food and dietary preparations.

It is of particular interest that the immuno-globulins should be non-specific, since this involves a large cost reduction, they have a therapeutic activity sufficient for slight infections and are an aid in more serious infections and do not mask any of the symptoms of the infection to be treated, whereby they may be used immediately such symptoms appear.

Finally, a further preferred application is obtained when their use is combined with the administration of antibiotics.

It is also an object of the invention to prepare a concentrate of the gamma globulin fraction of over 90 wt%, since in such case smaller amounts are required for the later applications thereof, by way of a process significantly reducing the production cost in comparison with the regular chromatographic techniques. This object is achieved with a process of the type first mentioned above, characterized in that: [i] said first concentrate is diluted in water in a first concentrate: water ratio ranging from 1:7 - 1:11, giving a first mixture; [ii] the pH of said first mixture is adjusted for the first time to a value ranging from 3 - 6, to give a second mixture; [iii] from 2 to 6 wt% of polyethylene glycol relative to said second mixture is added thereto, giving a third mixture; [iv] said third mixture is centrifuged for a first time at an acceleration ranging from 3000 to 11000 g, giving a first insolubilized beta globulin-rich fraction and a first clarified gamma globulin-rich fraction, both first fractions being separated; [v] the pH of said gamma globulin-rich fraction is adjusted for a second time to a value ranging from 6 to 9 to give a fourth mixture, to which polyethylene glycol is added to a weight proportion ranging from 6-12 wt% relative to said fourth mixture, to provide a fifth mixture; [vi] said fifth mixture is centrifuged for a second time at an acceleration ranging from 3000 - 11000 g to give a second insolubilized fraction composed mainly of gamma globulin and a second clarified fraction, both second fractions being separated.

The animal plasma used in this process is preferably porcine, bovine, ovine, equine or goat plasma, with the use of porcine plasma being particularly preferred.

Concentrates of the product to which the invention relates may be obtained with the use of spray drying driers, freeze-drying or any other drying technique. The present invention does not exclude the preparation of these concentrates in liquid or frozen form. They may also be prepared by microencapsulation of the active ingredient, protecting it and stabilizing it against high temperatures, oxidants, pH, light, humidity, etc.

They may be supplied in tablet, capsule, drinkable vial, granulated, powder, cream form, both as a sole ingredient and in association with other excipients or active ingredients.

### Detailed Description of the Invention

The invention consists of the application of immuno-globulin concentrates, particularly of porcine origin, for the prevention and co-treatment of gastrointestinal disorders of diverse origin. These concentrates are preferably of non-specific immuno-globulins. It is contemplated that they are administered orally or topically and are used both alone and in association with other substances.

The use of porcine immuno-globulins reduces the risk of allergy reactions caused by other immuno-globulin sources, having an allergenic risk. This allergenic risk appears, for example, due to the fact that both cow's milk and egg are foodstuffs used in infant nutrition in the first stages of replacement of the breast milk. This means that certain individuals have developed allergies specific to proteins from these sources.

The majority of processes in which specific immuno-globulins are used are costly, since animals must be hyperimmunized with specific antigens. These treatments require a precise prior diagnosis, which is costly both financially and in the time required for obtaining the results of the analyses.

The application of non-specific immuno-globulins (which contain antibodies for a very high number of microorganisms) is advantageous in light intestinal infections where the presence of antibodies available in the immuno-globulin concentrate is already sufficient to stop the symptoms caused by the infection. In this way, the symptoms may also be alleviated of more serious infections, in which it is necessary to have recourse to treatment with specific antibiotics after diagnosis, since they do not mask any of the symptoms of the infection and, therefore, may be applied immediately after the appearance of said symptoms.

The administration of non-specific immuno-globulins of porcine origin orally or enterally is particularly advantageous, since it does not cause immunological rejection problemssince the said immuno-globulins do not pass through the intestinal wall and since they perform their protective or therapeutic activity locally in the intestinal passage.

It frequently happens that, in population groups having a risk of gastrointestinal disorders, such as a new born babies of low weight at birth, new born babies not breast-feeding, elderly people with immuno deficiencies, adults having an impaired immunitary system, particularly in the case of AIDS, persons undergoing chemotherapy in cancer processes, or persons who have undergone a bone medulla transplant, gastrointestinal infections are chronic and are often accompanied by opportunistic infections taking advantage of the imbalance of the intestinal flora to manifest their pathogeny. In such cases, it usually happens that the therapies used are not completely effective, since in the best of cases they manage to reduce the effect caused by the first pathogen, but are inactive against the opportunistic germs, whereby the use of non-specific gamma globulin concentrates of broad antibacterial spectrum is very advisable in these situations, since further to helping to restore the bacterial ecosystem of the intestine, it prevents the action of opportunistic pathogens and thereby helps in the recovery of the intestinal epithelium. It is for this same reason that the joint application of an immuno-globulin enriched preparation with the specific therapy is of interest.

The application of porcine immuno-globulin enriched preparations affords the additional advantage that it does not cause the appearance of a secondary symptomatology associated with the use of other compounds which are used in processes of gastrointestinal disorders, such as are constipation, loss of gastrointestinal flora or reduction of appetite, since, with their being natural proteins, after their effect associated with the bioactivity of such molecules, they will be degraded or excreted in the same way as happens with the remaining animal proteins ingested daily in the diet.

Since it is a case of a high spectrum immuno-globulin concentrate, obtained from a very large volume of blood of animals from different sources, it is to be expected that it will contain antibodies for a very varied number of bacteria and virus. In human medicine, the treatment of viral gastrointestinal infections has not yet been solved, since the antibiotics do not have an effect on these virus, whereby the use of immuno-globulins could mean an advance in the reduction of the acuity of such infections.

It is well known that both bacteria and virus have the capacity of becoming resistant to antibiotics or chemical preparations. Mammals combat such resistance by creating new antibodies specific to the new mutation of the bacteria, in such a way that the bacterial or viral mutations are naturally corresponded to by the appearance of antibodies predominant in the blood against such mutations. In these cases, it usually happens that with time the antibiotics or immuno-globulins obtained from hyperimmunized animals cease to have a complete effect, and new generations of antibiotics or specific immuno-globulins have to be obtained. This does not happen in our case, since with the immuno-globulin enriched preparation being obtained from sacrificed animals, it contains antibodies against the new strains of bacteria that have mutated to acquire resistance to the antibiotics. In this way, they have at all times an immunological activity against the subclasses of pathogens which cause the gastrointestinal disorders.

These applications are also appropriate for pets. The microbiological quality of their food is lower than that of human food and above all is due to the fact that the food usually remains in the feeder for many hours at room temperature, whereby bacterial growth may develop under such conditions, and may cause intestinal infections. The use of specific immuno-globulins from hyperimmunized animals for the treatment of these gastrointestinal infections is infrequent because of the high cost involved in such therapy, as well as the lack of a precise diagnosis of such infection. Therefore, the use of broad bacterial and viral spectrum immuno-globulin concentrates, particularly those of porcine origin, is an economical and viable alternative for use in reducing the seriousness of gastrointestinal infections and recovering the animals' intestinal balance, as well as a preventive use in animals having chronic infections and, therefore, poor intestinal absorption.

It is a regular practice to include powdered plasma in the initiation and weaning diets of farm animals, particularly pigs. This affords benefits with regard to growth and reduction of diarrhoeas and mortality in this period of life. Such benefits have been associated in the scientific literature with the immuno-globulin fraction present in the powdered plasma, whereby the use of an immuno-globulin-rich fraction in the feeds administered at these ages will have a higher effect to that caused by the whole plasma. Pigs belong to a group of mammals born immunodeficient which need to ingest the maternal colostrum, rich in immuno-globulins, to incorporate passive immunity into their organism. This must occur during the first 24-36 hours of life, which is when there is an intestinal permeability to macromolecules, whereby part of the immuno-globulins ingested in the colostrum will pass into the sucking animal's blood, providing it with passive immunity for the first weeks, until the animal is capable of generating its own immunitary system.

Nevertheless, the supplies of immuno-globulins as substitutes for the maternal colostrum known on the market are based on bovine immuno-globulins. The fact of using porcine origin immuno-globulins represents an advantage over the use of bovine immuno-globulins, since the action time in blood of bovine immuno-globulins is substantially inferior to that of porcine immuno-globulins, whereby the passive immunity of the sucking animals is improved during the first 24 hours of life.

In some applications, it is convenient to have plasma protein mixtures containing gamma globulin concentrations of over 90%.

Nevertheless, in other applications, it is sufficient to have plasma protein mixtures containing gamma globulin concentrations, relative to total proteins, of over 25%. In these cases, said mixtures may be prepared in a cheaper way and, additionally, these mixtures retain other proteins, peptides, vitamins and growth factors of high value and which are removed in the plasma protein mixtures whose gamma globulin content is over 90%.

For a better understanding of the invention, there are disclosed hereinafter examples of the diverse steps of the process of the invention, with inclusion of steps prior to the preparation of the starting material.

### Example 1

### Separation of first fractions from animal plasma

1000 kg of unhaemolized porcine plasma were charged in a work reactor at 4°C and pH 7.7. Stirring was applied at 50 r.p.m. and 53.18 kg of hydrochloric acid were added, whereby the pH was lowered to 4.5. 55 kg of polyethylene glycol (PEG) 4000 were added, whereby the mixture contained 6 wt% of PEG. The pH was readjusted to 4.5. The reaction mixture was left under stirring for 5 hours and thereafter was centrifuged in a centrifuge at 9000 g, with a bowl for recovering the concentrated fraction therein. After centrifugation, 33.7 kg were obtained of a fibrinogen-rich fraction and 1052 kg of a clarified fraction containing the remaining soluble proteins.

After removal of the polyethylene glycol, the clarified fraction had a gamma globulin concentration, relative to total proteins, of approximately 30%.

### Example 2

### Treatment of the clarified fraction obtained

The 1052 kg of clarified fraction prepared according to Example 1 were held under constant stirring at 4°C and the pH was adjusted to 7.0 by addition of 33.32 kg of sodium hydroxide. Thereafter, 88.2 kg of PEG were added to maintain the wt% at 12, the pH being adjusted once again to 7.0. The mixture was left under constant stirring at the temperature indicated for 5 hours. Thereafter, the mixture was centrifuged in the same centrifuge as mentioned in the previous example and 1093 kg of a new clarified fraction and 54.8 kg of a gamma globulin-rich concentrate were obtained.

This gamma globulin-rich concentrate had a gamma globulin concentration relative to total proteins of approximately 50%.

### Example 3

### Treatment of the gamma globulin-rich concentrate

To the 54.8 kg of gamma globulin-rich concentrate obtained according to the previous example were added 493 kg of water (concentrate:water ratio → 1:9). The pH was adjusted to 6.0 by addition of 7.7 kg of hydrochloric acid and a further 32.8 kg of PEG were added to keep the PEG concentration at 6 wt%, the pH being adjusted again with hydrochloric acid. The mixture was held under constant stirring at 4°C for 5 hours, after which it was centrifuged in a centrifuge of the type indicated in the previous examples. After the centrifugation process, 17 kg were obtained of a beta globulin-rich insolubilized fraction and 552 kg of a clarified gamma globulin-rich fraction.

### Example 4

### Treatment of the new clarified fraction obtained

To the 552 kg of the clarified gamma globulin-rich fraction obtained according to the previous example there were added 4.6 kg of sodium hydroxide, whereby the pH thereof was adjusted to 9.0. Furthermore, 38 kg of PEG were added, whereby the proportion of PEG (bearing in mind the PEG contained in the fraction) was held at 12 wt%. The pH was adjusted again to 9.0. The mixture was held under constant stirring at 4°C for 5 hours, after which it was centrifuged in an apparatus of the type mentioned above and thus 550 kg of a clarified fraction and 30 kg of a fraction having a high gamma globulin content were obtained.

### Example 5

### Treatment of the high gamma globulin content fraction

The 30 kg of the high gamma globulin content fraction obtained according to the previous example were dissolved in water by addition of 60 kg of water (ratio 1:2 by weight). The pH was adjusted to 6.0. Once the mixture was perfectly dissolved, it was dried in an atomizer (spray dried), by removing the water contained in the liquid product by subjecting it to transportation with high temperature air (150°C at the inlet and 100°C at the outlet) for a short period of time, whereby the water was evaporated without affecting the solubility of the protein to be dried. The powdered product was finally packaged. The characteristics of the powdered product obtained were:

| | |
|---|---|
| Humidity | < 10 % |
| Protein | 90 ± 2% |
| Ashes | < 2% |
| Insolubles | < 2% |
| Standard proteinogram % (relative to protein) | |
| Albumin | 1.0 % |
| Alpha globulin | 5.3 % |
| Beta globulin | 4.0 % |
| Gamma globulin | 90.63 % |

The utility of the compositions to which reference is made may be due in part to the fact that the product prepared according to the process of the invention may contain IgM, transferrin, cellular growth factors or alpha 2 macroglobulin.

## Claims

1. Applications of mixtures of gamma globulin-rich plasma proteins of porcine origin having a gamma globulin content of over 25 wt%, relative to total proteins, for the preparation of medicinal compositions, food and dietary preparations suitable for the prevention and treatment of gastrointestinal disorders in human beings.

2. Applications of mixtures of gamma globulin-rich plasma proteins of porcine origin having a gamma globulin content of over 25 wt%, relative to total proteins, for the preparation of medicinal compositions, food and dietary preparations suitable for the treatment of malabsorption processes in human beings.

3. Applications of mixtures of gamma globulin-rich plasma proteins of non human animal origin having a gamma globulin content of over 25 wt%, relative to total proteins, for the preparation of medicinal compositions, food and dietary preparations suitable for the treatment of a misabsorption process from the group formed by: small intestine syndrome; refractory diarrhoea of autoimmune origin; lymphoma; post-gastrectomy steatorrhea; carcinoma of the pancreas; wide pancreatic resection; mesenteric vascular insufficiency; amyloidosis; sclerodermia; eosinophilic enteritis.

4. Applications of mixtures of gamma globulin-rich plasma proteins of porcine origin having a gamma globulin content of over 25 wt%, relative to total proteins, for the preparation of medicinal compositions, food and dietary preparations suitable for the treatment of clinical conditions with malnutrition in human beings.

5. The application according to claim 4, **characterized in that** said clinical conditions are: ulcerative colitis; Crohn's disease; cancerous cachexia for chronic enteritis caused by chemo- and/or radiotherapy; infectious medical pathology involving severe malabsorption; AIDS; cystic fibrosis; low flow enterocutaneous fistulas; and infantile renal insufficiency endangering patient growth.

6. The application according to claim 3, **characterized in that** said plasma proteins are obtained from porcine, bovine, goat, equine or ovine plasma.

7. The application according to at least one of claims 1 to 6, **characterized in that** said medicinal compositions, food and dietary preparations may be used dissolved in water, milk, juices, yoghurt or other foodstuffs.

8. The application according to claim 7, **characterized in that** said other foodstuffs are humanised milk, paps or other baby foods.

9. The application according to at least one of claims 1 to 8, **characterized in that** to said medicinal compositions, food and dietary preparations there may be added any variety of fibres, prebiotic, probiotic products, vitamins or minerals.

10. The application according to at least one of claims 1 to 9, **characterized in that** said medicinal compositions, food and dietary preparations are preferably used with the administration of antibiotics.

11. The application according to at least one of claims 6 to 10, **characterized in that** said mixtures of plasma proteins are of porcine origin and **in that** they are applied for the treatment of human beings.

12. The application according to at least one of claims 1 to 11, **characterized in that** said gamma globulin content is over 90 wt%, relative to total proteins.

13. A process for the preparation of mixtures of plasma proteins of non-human animal origin having a gamma globulin content of over 90 wt%, relative to total proteins, without the use of chromatographic columns, from a first concentrate, the gamma globulin/proteins ratio of which is over 30 wt%, said first concentrate being derived from animal plasma which has been subjected to at least one prior centrifugation operation, **characterized in that**: [i] said first concentrate is diluted in water in a first concentrate: water ratio ranging from 1:7 - 1:11, giving a first mixture; [ii] the pH of said first mixture is adjusted for the first time to a value ranging from 3 - 6, to give a second mixture; [iii] from 2 to 6 wt% of polyethylene glycol relative to said second mixture is added thereto, giving a third mixture; [iv] said third mixture is centrifuged for a first time at an acceleration ranging from 3000 to 11000 g, giving a first insolubilized beta globulin-rich fraction and a first clarified gamma globulin-rich fraction, both first fractions being separated; [v] the pH of said gamma globulin-rich fraction is adjusted for a second time to a value ranging from 6 to 9 to give a fourth mixture, to which polyethylene glycol is added to a weight proportion ranging from 6-12 wt% relative to said fourth mixture, to provide a fifth mixture; [vi] said fifth mixture is centrifuged for a second time at an acceleration ranging from 3000 - 11000 g to give a second insolubilized fraction composed mainly of gamma globulin and a second clarified fraction, both second fractions being separated.

14. The process according to claim 13, **characterized in that** said animal plasma is porcine, bovine, ovine, equine or goat plasma.

15. The process according to claim 14, **characterized in that** said animal plasma is porcine plasma.

16. The process according to at least one of claims 13 to 15, **characterized in that** said second insolublized fraction is dissolved in water at a second insolublized fraction/water ratio ranging from 1:2 - 1:9, the pH is adjusted for a third time to a value ranging from 5 to 9 and the fraction is dried.

17. The process according to claim 16, **characterized in that** said drying is effected in an atomizer, said second insolubilized fraction being subjected to a high temperature air flow.

18. The process according to claim 17, **characterized in that** said high temperature varies over a range of 150-250°C at the inlet and a range of 70-100°C at the outlet.

19. The process according to at least one of claims 13 to 16, **characterized in that** said gamma globulin concentrates are prepared in liquid, frozen, concentrated or dried form by a process other than the one using an atomizer.

20. The process according to at least one of claims 13 to 19, **characterized in that** said first adjustment is effected with hydrochloric or phosphoric acid.

21. The process according to at least one of claims 13 to 20, **characterized in that** said separation is effected in a filter press with a filtering pore sieve.

22. The process according to at least one of claims 13 to 17, **characterized in that** said separation is effected in a centrifuge having an acceleration of over 3000 g.

23. The process according to claim 22, **characterized in that** said centrifuge is continuous.

24. The process according to claim 22, **characterized in that** said centrifuge has an internal bowl.

25. The process according to at least one of claims 13 to 22, **characterized in that** said second adjustment is effected with sodium hydroxide.

## Patentansprüche

1. Verwendung von Gemischen von Gammaglobulin-reichen Plasmaproteinen aus Schwein mit einem Gammaglobulin-Anteil von über 25 Gew.-%, bezogen auf das Gesamtprotein, für die Herstellung von medizinischen Zusammensetzungen, Nahrungsmittelzubereitungen oder diätetischen Zubereitungen, die für die Verhinderung oder Behandlung von gastrointestinalen Störungen im Menschen geeignet sind.

2. Verwendung von Gemischen von Gammaglobulin-reichen Plasmaproteinen aus Schwein mit einem Gammaglobulin-Anteil von über 25 Gew.-%, bezogen auf das Gesamtprotein, für die Herstellung von medizinischen Zusammensetzungen, Nahrungsmittelzubereitungen oder diätetischen Zubereitungen, die für die Behandlung von Malabsorptionsvorgängen im Menschen geeignet sind.

3. Verwendung von Gemischen von Gammaglobulin-reichen Plasmaproteinen nicht-menschlichen tierischen Ursprungs mit einem Gammaglobulin-Anteil von über 25 Gew.-%, bezogen auf das Gesamtprotein, für die Herstellung von medizinischen Zusammensetzungen, Nahrungsmittelzubereitungen oder diätetischen Zubereitungen, die für die Behandlung von Malabsorptionsvorgängen geeignet sind, die aus der folgenden Gruppe ausgewählt sind: Dünndarmsyndrom; hartnäckiger Durchfall autoimmunogenen Ursprungs; Lymphoma; Fettstuhl nach einer Magenresektion; Bauchspeicheldrüsenkarzinom; umfangreiche Pankreasresektion; Insuffizienz der mesenterischen Gefäße, Amyloidosis; Hautentzündung; eosinophile Darmerkrankung.

4. Verwendung eines Gemisches von Gammaglobulin-reichen Plasmaproteinen aus Schwein mit einem Gammaglobulin-Anteil von über 25 Gew.-%, bezogen auf das Gesamtprotein, für die Herstellung von medizinischen Zusammensetzungen, Nahrungsmittelzubereitungen und diätetischen Zubereitungen, die für die Behandlung von klinischen Zuständen mit Fehlemährung im Menschen geeignet sind.

5. Verwendung nach Anspruch 4, **dadurch gekennzeichnet, daß** die klinischen Zustände sind: ulceröse Kolitis; Morbus Crohn; Krebscachexie bei chronischer Darmentzündung, verursacht durch Chemo- und/oder Radiotherapie; infektiöse medizinische Befunde, die schwere Malabsorption betreffen; Aids; zystische Fibriose; schwach fließende enterokutane Fisteln; und infantile Niereninsuffizienz, welche das Wachstum des Patienten gefährdet.

6. Verwendung nach Anspruch 3, **dadurch gekennzeichnet, daß** die Plasmaproteine aus Schweine-, Rinder-, Ziegen-, Pferde- oder Eierplasma erhalten werden.

7. Verwendung nach einem der Ansprüche 1 bis 6, **dadurch gekennzeichnet, daß** die medizinischen Zusammensetzungen, die Nahrungsmittelzubereitungen und diätetischen Zubereitungen gelöst in Wasser, Milch, Säften, Joghurt oder anderen Nahrungsmitteln eingesetzt werden können.

8. Verwendung nach Anspruch 7, **dadurch gekennzeichnet, daß** die anderen Nahrungsmittel auf menschliche Bedürfnisse eingestellte Milch, Brei oder andere Babynahrungsmittel sind.

9. Verwendung nach einem der Ansprüche 1 bis 8, **dadurch gekennzeichnet, daß** die medizinischen Zusammensetzungen, die Nahrungsmittelzubereitungen und diätetischen Zubereitungen mit beliebigen verschiedenen Ballaststoffen, präbiotischen, probiotischen Produkten, Vitaminen oder Mineralien versetzt werden können.

10. Verwendung nach einem der Ansprüche 1 bis 9, **dadurch gekennzeichnet, daß** die medizinischen Zusammensetzungen, die Nahrungsmittelzubereitungen und diätetischen Zubereitungen vorzugsweise mit der Verabreichung von Antibiotika eingesetzt werden.

11. Verwendung nach einem der Ansprüche 6 bis 10, **dadurch gekennzeichnet, daß** die Gemische von Plasmaproteinen aus Schwein stammen und daß sie für die Behandlung des Menschen eingesetzt werden.

12. Verwendung nach einem der Ansprüche 1 bis 11, **dadurch gekennzeichnet, daß** der Gammaglobulin-Anteil über 90 Gew.-%, bezogen auf das Gesamtprotein, ist.

13. Verfahren zur Herstellung eines Gemisches von Plasmaproteinen nicht-menschlichen tierischen Ursprungs mit einem Gammaglobulin-Anteil von über 90 Gew.-%, bezogen auf das Gesamtprotein, ohne die Verwendung von chromatographischen Säulen aus einem ersten Konzentrat mit einem Gammaglobulin/Protein-Verhältnis von über 30 Gew.-%, wobei das erste Konzentrat von tierischem Plasma abgeleitet ist, das mindestens einer vorherigen Zentrifugation unterworfen worden ist, **dadurch gekennzeichnet, daß**: [i] das erste Konzentrat in Wasser in einem ersten Konzentrat/Wasser-Verhältnis im Bereich von 1:7 bis 1:11 verdünnt wird, wobei ein erstes Gemisch erhalten wird; [ii] der pH-Wert des ersten Gemisches zum ersten Mal auf einen Wert im Bereich von 3 bis 6 eingestellt wird, wobei ein zweites Gemisch erhalten wird; [iii] von 2 bis 6 Gew.-% Polyethylenglycol, bezogen auf das zweite Gemisch, zugegeben wird, wobei ein drittes Gemisch erhalten wird; [iv] das dritte Gemisch zum ersten Mal bei einer Beschleunigung im Bereich von 3000 bis 11 000 g zentrifugiert wird, wobei eine erste reiche Fraktion an unlöslich gemachtem β-Globulin und eine erste reiche Fraktion an klarem Gammaglobulin erhalten wird, wobei beide ersten Fraktionen getrennt werden; [v] der pH-Wert der Gammaglobulin-reichen Fraktion zum zweiten Mal auf einen Wert im Bereich von 6 bis 9 eingestellt wird, wobei ein viertes Gemisch erhalten wird, zu dem Polyethylenglycol in einem Gewichtsanteil im Bereich von 6 bis 12 Gew.-%, bezogen auf das vierte Gemisch, gegeben wird, wobei ein fünftes Gemisch erhalten wird; [vi] das fünfte Gemisch zum zweiten Mal bei einer Beschleunigung im Bereich von 3000 bis 11 000 g zentrifugiert wird, wobei eine zweite unlöslich gemachte Fraktion, die hauptsächlich aus Gammaglobulin besteht, und eine zweite klare Fraktion erhalten werden, wobei beide zweiten Fraktionen getrennt werden.

14. Verfahren nach Anspruch 13, **dadurch gekennzeichnet, daß** das tierische Plasma Schweine-, Rinder-, Eier-, Pferde- oder Ziegenplasma ist.

15. Verfahren nach Anspruch 14, **dadurch gekennzeichnet, daß** das tierische Plasma Schweineplasma ist.

16. Verfahren nach einem der Ansprüche 13 bis 15, **dadurch gekennzeichnet, daß** die zweite unlöslich gemachte Fraktion in Wasser bei einem Verhältnis der zweiten unlöslichen Fraktion zu Wasser im Bereich von 1:2 bis 1:9 aufgelöst wird, der PH-Wert zum dritten Mal auf einen Wert im Bereich von 5 bis 9 eingestellt wird, und die Fraktion getrocknet wird.

17. Verfahren nach Anspruch 16, **dadurch gekennzeichnet, daß** das Trocknen in einem Zerstäuber durchgeführt wird, wobei die zweite unlösliche Fraktion einem Hochtemperatur-Luftstrom unterworfen wird.

18. Verfahren nach Anspruch 17, **dadurch gekennzeichnet, daß** die Hochtemperatur in einem Bereich von 150 bis 250°C am Einlaß und im Bereich von 709 bis 100°C am Auslaß variiert.

19. Verfahren nach einem der Ansprüche 13 bis 16, **dadurch gekennzeichnet, daß** die Gammaglobulin-Konzentrate in flüssiger, gefrorener, konzentrierter oder getrockneter Form durch ein Verfahren hergestellt werden, das nicht das Verfahren unter Einsatz eines Zerstäubers ist.

20. Verfahren nach einem der Ansprüche 13 bis 19, **dadurch gekennzeichnet, daß** die erste Einstellung mit Chlorwasserstoffsäure oder Phosphorsäure bewirkt wird.

21. Verfahren nach einem der Ansprüche 13 bis 20, **dadurch gekennzeichnet, daß** die Trennung in einer Filterpresse mit einem Filterporensieb durchgeführt wird.

22. Verfahren nach einem der Ansprüche 13 bis 17, **dadurch gekennzeichnet, daß** die Trennung in einer Zentrifuge mit einer Beschleunigung von über 3000 g bewirkt wird.

23. Verfahren nach Anspruch 22, **dadurch gekennzeichnet, daß** die Zentrifuge kontinuierlich ist.

24. Verfahren nach Anspruch 22, **dadurch gekennzeichnet, daß** die Zentrifuge einen internen Schleuderraum hat.

25. Verfahren nach einem der Ansprüche 13 bis 22, **dadurch gekennzeichnet, daß** die zweite Einstellung mit Natriumhydroxid bewirkt wird.

## Revendications

1. Utilisations de mélanges de protéines plasmatiques riches en gamma globulines d'origine porcine présentant une teneur en gamma globulines supérieure à 25 % en poids, par rapport aux protéines totales, pour la préparation de compositions thérapeutiques, de préparations alimentaires et diététiques pour la prévention et le traitement de troubles gastro-intestinaux chez les êtres humains.

2. Utilisations de mélanges de protéines plasmatiques riches en gamma globulines d'origine porcine présentant une teneur en gamma globulines supérieure à 25 % en poids, par rapport aux protéines totales, pour la préparation de compositions thérapeutiques, de préparations alimentaires et diététiques convenant au traitement de processus de malabsorption chez les êtres humains.

3. Utilisations de mélanges de protéines plasmatiques riches en gamma globulines d'origine animale non humaine présentant une teneur en gamma globulines supérieure à 25 % en poids, par rapport aux protéines totales, pour la préparation de compositions thérapeutiques, de préparations alimentaires et diététiques convenant au traitement d'un processus de mauvaise absorption compris dans le groupe formé par : le syndrome de l'intestin grêle ; la diarrhée réfractaire d'origine auto-immune ; le lymphome ; la stéatorrhée post-gastrectomie ; le carcinome du pancréas ; une résection considérable du pancréas ; l'insuffisance vasculaire mésentérique ; l'amyloïdose ; la sclérodermie ; et l'entérite éosinophile.

4. Utilisations de mélanges de protéines plasmatiques riches en gamma globulines d'origine porcine présentant une teneur en gamma globulines supérieure à 25 % en poids, par rapport aux protéines totales, pour la préparation de compositions thérapeutiques, de préparations alimentaires et diététiques convenant au traitement d'états cliniques associés à la malnutrition chez les êtres humains.

5. Utilisation selon la revendication 4, **caractérisée en ce que** lesdits états cliniques sont : la colite ulcéreuse, la maladie de Crohn ; la cachexie tumorale due à une entérite chronique provoquée par chimio- et/ou radiothérapie ; une pathologie médicale infectieuse impliquant une malabsorption grave ; le SIDA ; la fibrose cystique ; les fistules entérocutanées à faible débit ; et l'insuffisance rénale infantile consituant une menace pour la croissance du patient.

6. Utilisation selon la revendication 3, **caractérisée en ce que** lesdites protéines plasmatiques sont obtenues à partir de plasma porcin, bovin, de chèvre, équin ou ovin.

7. Utilisation selon au moins l'une des revendications 1 à 6, **caractérisée en ce que** lesdites compositions thérapeutiques, préparations alimentaires et diététiques peuvent être utilisées dissoutes dans de l'eau, du lait, des jus, des yoghourts ou d'autres denrées alimentaires.

8. Utilisation selon la revendication 7, **caractérisée en ce que** lesdites autres denrées alimentaires sont du lait matemisé, des bouillies et d'autres aliments pour bébés.

9. Utilisation selon au moins l'une des revendications 1 à 8, **caractérisée en ce qu'**aux dites compositions thérapeutiques, préparations alimentaires et diététiques peut être ajoutée n'importe quelle variété de fibres, de produits prébiotiques, probiotiques, de vitamines et de minéraux.

10. Utilisation selon au moins l'une des revendications 1 à 9, **caractérisée en ce que** lesdites compositions thérapeutiques, préparations alimentaires et diététiques sont utilisées de préférence accompagnées de l'administration d'antibiotiques.

11. Utilisation selon au moins l'une des revendications 6 à 10, **caractérisée en ce que** lesdits mélanges de protéines plasmatiques sont d'origine porcine et **en ce qu'**ils sont utilisés pour le traitement d'êtres humains.

12. Utilisation selon au moins l'une des revendications 1 à 11, **caractérisée en ce que** ladite teneur en gamma globulines est supérieure à 90 %, par rapport aux protéines totales.

13. Procédé pour la préparation de mélanges de protéines plasmatiques d'origine animale non humaine présentant une teneur en gamma globulines supérieure à 90 %, par rapport aux protéines totales, sans l'utilisation de colonnes de chromatographie, à partir d'un premier concentré, dont le rapport gamma globulines/protéines est supérieur à 30 %, ledit premier concentré provenant de plasma animal qui a été soumis à au moins une opération de centrifugation préalable, **caractérisé en ce que** :[i] ledit premier concentré est dilué dans de l'eau selon un premier concentré : rapport eau compris entre 1 : 7 et 1 : 11, pour donner un premier mélange ; [ii] le pH dudit premier mélange est ajusté pour la première fois à une valeur comprise entre 3 et 6, pour donner un deuxième mélange ; [iii] entre 2 et 6 % en poids de polyéthylène glycol par rapport audit deuxième mélange sont ajoutés à celui-ci, pour donner un troisième mélange ; [iv] ledit troisième mélange est centrifugé pour une première fois à une accélération comprise entre 3000 et 11000 g, pour donner une première fraction insolubilisée riche en bêta globulines et une première fraction clarifiée riche en gamma globulines, les deux premières fractions étant séparées ; [v] le pH de ladite fraction riche en gamma globulines est ajusté pour une deuxième fois à une valeur comprise entre 6 et 9 pour fournir un quatrième mélange, auquel est ajouté du polyéthylène glycol jusqu'à une proportion en poids comprise entre 6 et 12 % en poids par rapport audit quatrième mélange, afin de fournir un cinquième mélange ; [vi] ledit cinquième mélange est centrifugé pour une deuxième fois à une accélération comprise entre 3000 et 11000 g pour fournir une seconde fraction insolubilisée se composant principalement de gamma globulines et une seconde fraction clarifiée, les deux secondes fractions étant séparées.

14. Procédé selon la revendication 13, **caractérisé en ce que** ledit plasma animal est du plasma porcin, bovin, ovin, équin ou de chèvre.

15. Procédé selon la revendication 14, **caractérisé en ce que** ledit plasma animal est du plasma porcin.

16. Procédé selon au moins l'une des revendications 13 à 15, **caractérisé en ce que** ladite seconde fraction insolubilisée est dissoute dans de l'eau selon un rapport seconde fraction insolubilisée/eau compris entre 1 : 2 et 1 : 9, le pH est ajusté pour une troisième fois à une valeur comprise entre 5 et 9 et la fraction est séchée.

17. Procédé selon la revendication 16, **caractérisé en ce que** ledit séchage s'effectue dans un atomiseur, ladite seconde fraction insolubilisée étant soumise à un flux d'air à température élevée.

18. Procédé selon la revendication 17, **caractérisé en ce que** ladite température élevée varie dans un intervalle compris entre 150 et 250 °C à l'entrée et un intervalle compris entre 70 et 100 °C à la sortie.

19. Procédé selon au moins l'une des revendications 13 à 16, **caractérisé en ce que** lesdits concentrés de gamma globulines sont préparés sous forme liquide, congelée, concentrée ou desséchée par un procédé autre que celui utilisant un atomiseur.

20. Procédé selon au moins l'une des revendications 13 à 19, **caractérisé en ce que** ledit premier ajustement s'effectue à l'aide d'acide chlorhydrique ou phosphorique.

21. Procédé selon au moins l'une des revendications 13 à 20, **caractérisé en ce que** ladite séparation s'effectue dans un filtre-presse muni d'un tamis à pores filtrants.

22. Procédé selon au moins l'une des revendications 13 à 17, **caractérisé en ce que** ladite séparation s'effectue dans une centrifugeuse possédant une accélération supérieure à 3000 g.

23. Procédé selon la revendication 22, **caractérisé en ce que** ladite centrifugeuse est continue.

24. Procédé selon la revendication 22, **caractérisé en ce que** ladite centrifugeuse est munie d'une cuve interne.

25. Procédé selon au moins l'une des revendications 13 à 22, **caractérisé en ce que** ledit deuxième ajustement s'effectue à l'aide d'hydroxyde de sodium.
